(19) Europäisches Patentamt
European Patent Office
Office européen des brevets

(11) **EP 1 587 777 B1**

(12) **FASCICULE DE BREVET EUROPEEN**

(45) Date de publication et mention de la délivrance du brevet:
**19.03.2008 Bulletin 2008/12**

(21) Numéro de dépôt: **04703173.7**

(22) Date de dépôt: **19.01.2004**

(51) Int Cl.:
**C07C 11/18** (2006.01)   **C10G 70/00** (2006.01)
**C08F 136/08** (2006.01)

(86) Numéro de dépôt international:
**PCT/EP2004/000343**

(87) Numéro de publication internationale:
**WO 2004/065339 (05.08.2004 Gazette 2004/32)**

(54) **PROCEDE D'OBTENTION D'UNE COUPE C5   FCC ENRICHIE EN ISOPRENE ET POLYMERISATION SELECTIVE DE L'ISOPRENE A PARTIR DE CETTE COUPE**

VERFAHREN ZUR GEWINNUNG EINERISOPRENANGEREICHERTEN FCC-C5-FRAKTION UND SELEKTIVE POLYMERISIERUNG VONISOPREN AUS DIESER FRAKTION

METHOD OF OBTAINING AN ISOPRENE-ENRICHED FCC C5 FRACTION AND SELECTIVE POLYMERISATION OF ISOPRENE FROM SAID FRACTION

(84) Etats contractants désignés:
**AT BE BG CH CY CZ DE DK EE ES FI FR GB GR HU IE IT LI LU MC NL PT RO SE SI SK TR**

(30) Priorité: **22.01.2003   FR 0300694**

(43) Date de publication de la demande:
**26.10.2005   Bulletin 2005/43**

(73) Titulaires:
• **Société de Technologie Michelin
  63000 Clermont-Ferrand Cedex 09 (FR)**
• **Michelin Recherche et Technique S.A.
  1763 Granges-Paccot (CH)**

(72) Inventeur: **GANDON-PAIN, Sylvie
  F-63100 Clermont-Ferrand (FR)**

(74) Mandataire: **Hiebel, Robert
  Michelin & Cie,
  Service SGD/LG/PI Ladoux
  63040 Clermont-Ferrand Cedex 01 (FR)**

(56) Documents cités:
  **WO-A-02/48218**          **FR-A- 1 203 754**

**Description**

La présente invention concerne un procédé d'obtention, à partir d'une coupe C5 de craquage catalytique sur lit fluide, d'une coupe C5 enrichie en isoprène et purifiée qui est utilisable pour former un milieu pour la polymérisation sélective de l'isoprène. L'invention concerne également un procédé d'obtention d'un homopolymère d'isoprène présentant notamment un taux d'enchaînements cis-1,4 et une viscosité inhérente élevés, à partir d'un milieu de polymérisation comprenant de l'isoprène et au moins un méthyl butène, tel que ladite coupe C5 de craquage catalytique sur lit fluide enrichie en isoprène et purifiée.

Les coupes C5 de craquage catalytique sur lit fluide (encore appelées coupes C5 d'essences légères de craquage catalytique sur lit fluide, ou en abrégé coupes F. C. C. pour « fluid catalytic cracking », en anglais) contiennent usuellement de l'isoprène selon une fraction massique inférieure à 1 %. En outre, elles contiennent essentiellement :

- selon une fraction massique typiquement comprise entre 40 % et 65 %, des mono-oléfines comprenant, d'une part, des $\alpha$-oléfines telles que butène-1, le méthyl-3 butène-1, le pentène-1, le méthyl-2 butène-1 et, d'autre part, des $\beta$-oléfines telles que le butène-2, le pentène-2 et le méthyl-2-butène-2 ;
- selon une fraction massique typiquement comprise entre 55 % et 30 %, des alcanes comprenant de l'isopentane à titre majoritaire et du n-pentane à titre minoritaire, et
- selon une fraction massique typiquement inférieure à 1 % , des diènes tels que le cyclopentadiène, le pentadiène-1,3 et le pentadiène-1,4, et d'autres composés tels que des composés acétyléniques.

Ces coupes C5 de craquage catalytique sur lit fluide ne doivent en particulier pas être confondues avec des coupes C5 de vapocraquage de naphta, qui contiennent typiquement de l'isoprène selon une fraction massique allant de 10 % à 30 %, des mono-oléfines ($\alpha$-oléfines et $\beta$-oléfines) selon une fraction massique allant de 20 % à 40 %, des diènes tels que le cyclopentadiène et des pentadiènes selon une fraction massique allant de 20 % à 30 %, et, à titre minoritaire, des alcanes, du limonène et des composés acétyléniques et aromatiques.

Pour pouvoir procéder à la polymérisation sélective de l'isoprène avec une activité élevée à partir d'une telle coupe C5 de vapocraquage de naphta, on doit au préalable enrichir cette dernière en isoprène pour que la fraction massique d'isoprène dans la coupe enrichie soit proche de 100 %. En effet, il s'avère que les autres composés précités nuisent au rendement de la réaction de polymérisation de l'isoprène. En particulier, cette coupe enrichie doit être pratiquement exempte de cyclopentadiène, qui est un poison pour les systèmes catalytiques.

Le document de brevet international WO-A-02/48218 au nom des demanderesses divulgue un procédé d'obtention d'un polyisoprène présentant un taux très élevé d'enchaînements cis-1,4 à partir d'une telle coupe C5 de vapocraquage de naphta enrichie en isoprène, ce procédé consistant essentiellement à faire réagir un système catalytique en présence de la coupe C5 enrichie de telle sorte que la fraction massique d'isoprène dans la coupe enrichie varie d'une manière surprenante de 30 % à 95 % seulement.

Ce système catalytique est à base d'un monomère diène conjugué, d'un sel de terre rare d'un acide phosphorique organique en suspension dans un solvant hydrocarboné inerte, saturé et de type aliphatique ou alicyclique, d'un agent d'alkylation constitué d'un alkylaluminium de formule $AlR_3$ ou $HAlR_2$, dans laquelle A1 représente l'atome d'aluminium, H représente un atome d'hydrogène et R représente un radical hydrocarboné, selon un rapport molaire (agent d'alkylation/ sel de terre rare) variant de 1 à 5, et d'un donneur d'halogène constitué d'un halogénure d'alkylaluminium.

Le document FR 1 203 754 décrit un procédé de préparation d'isoprène sensiblement pur à partir d'hydrocarbures acycliques en C5. Ce procédé, relativement long, comprend, dans une première étape, une déshydrogénation d'une fraction riche en alcanes et d'une recycle composée de monooléfines. On obtient alors trois flux, l'un composé d'hydrogène et d'hydrocarbure en moins de C5, le deuxième composé de diènes en C5 et le troisième de monooléfines en C5. On procède à une séparation de ces flux. Le procédé comprend ensuite une deuxième séparation pour isoler l'isoprène sensiblement pur des autres diènes en C5. Dans une autre étape, le procédé comprend une hydrogénation des diènes pour obtenir principalement de monooléfines normales qui sont ensuite isomérisées. On procède finalement au recyclage des isooléfines obtenues avec une fraction du troisième flux contenant des monooléfines dans l'unité de déshydrogénation de départ.

Pour pouvoir procéder à la polymérisation sélective de l'isoprène avec une activité élevée à partir d'une coupe C5 de craquage catalytique sur lit fluide, on doit également enrichir cette coupe C5 en isoprène pour que la fraction massique d'isoprène dans la coupe enrichie soit proche de 100 %, typiquement supérieure à 99 %. En effet, il s'avère que certains composés de cette coupe C5 enrichie, tels que les méthyl butènes, nuisent au rendement de la réaction de polymérisation de l'isoprène. De plus, la coupe enrichie doit être pratiquement exempte de pentadiène-1,3 et -1,4, connus pour pénaliser la cinétique de polymérisation de l'isoprène en comparaison de celle de l'isoprène pris isolément.

D'une manière connue, cette obtention d'une fraction massique d'isoprène proche de 100 % dans une coupe C5 de craquage catalytique sur lit fluide peut être obtenue, dans une première étape, par décomposition d'un éther amylique obtenu par réaction d'un alcool avec cette coupe C5 pour obtenir une fraction massique de méthyl butènes proche de 100 % dans la coupe C5 ainsi traitée et purifiée, puis, dans une seconde étape, par déshydrogénation oxydante des méthyl butènes en isoprène. On se reportera au document de brevet FR-A-2 782 996 pour la description de cet enrichissement en isoprène d'une coupe C5 « F. C. C. ».

Un inconvénient majeur des procédés connus de polymérisation sélective de l'isoprène à partir d'une coupe C5 de craquage catalytique sur lit fluide réside dans la nécessité d'enrichir considérablement en isoprène cette coupe C5 via un procédé d'enrichissement complexe tel que celui précité en référence au document FR-A-2 782 996 et, par conséquent, d'impliquer un coût opératoire global relativement élevé pour cette polymérisation.

Le but de la présente invention est de pallier cet inconvénient, et il est atteint en ce que les demanderesses ont découvert d'une manière surprenante qu'un système catalytique à base:

- d'au moins un monomère diène conjugué,
- d'un sel d'un ou plusieurs métaux de terre rare (métaux ayant un numéro atomique compris entre 57 et 71 dans le tableau de Mendeleev) d'un acide phosphorique organique,
- d'un agent d'alkylation constitué d'un alkylaluminium de formule $AlR_3$ ou $HAlR_2$, dans laquelle Al représente l'atome d'aluminium, H représente un atome d'hydrogène, les radicaux R, identiques ou différents, linéaires ou ramifiés, représentent des groupes hydrocarbonés ayant de 1 à 8 atomes de carbone, et
- d'un donneur d'halogène constitué d'un halogénure d'alkylaluminium,

ledit sel étant en suspension dans au moins un solvant hydrocarboné inerte, saturé et de type aliphatique ou alicyclique qui est compris dans ledit système catalytique, et le rapport molaire (agent d'alkylation / sel de terre rare) appartenant à un domaine allant de 1 à 5,
permet la polymérisation sélective de l'isoprène avec une activité catalytique élevée pour l'obtention d'un polyisoprène à taux élevé d'enchaînements cis-1,4, à partir d'une coupe C5 initiale de craquage catalytique sur lit fluide qui n'a été que peu enrichie en isoprène, de sorte que la fraction massique d'isoprène dans la coupe C5 finale enrichie et purifiée soit inférieure à 30 % et, avantageusement, inférieure ou égale à 10 %.

Il résulte de cette possibilité d'homopolymériser l'isoprène à partir de cette coupe C5 finale purifiée, très peu enrichie en isoprène, une simplification de l'opération d'enrichissement de la coupe C5 initiale et, par conséquent, un abaissement substantiel du coût global d'obtention des polyisoprènes à partir de ladite coupe C5 initiale.

Selon un autre aspect de la présente invention, un procédé d'obtention de ladite coupe C5 finale enrichie en isoprène et purifiée qui est utilisable pour former un milieu pour la polymérisation sélective de l'isoprène en présence dudit système catalytique, à partir de ladite coupe C5 initiale de craquage catalytique sur lit fluide, comprend :

- une déshydrogénation qui est appliquée à une coupe C5 intermédiaire, comprenant des méthyl butènes et issue de ladite coupe C5 initiale, et qui produit ladite coupe C5 finale,
- une purification de ladite coupe C5 finale ainsi obtenue pour l'obtention de ladite coupe C5 finale et purifiée qui soit pratiquement exempte d'alcynes disubstitués, d'alcynes vrais (y compris les vinylacétyléniques) et de cyclopentadiène,

et ce procédé d'obtention de ladite coupe C5 finale et purifiée est tel que la fraction massique desdits méthyl butènes dans ladite coupe C5 intermédiaire est inférieure à 30 %.

Avantageusement, la fraction massique desdits méthyl butènes dans ladite coupe C5 intermédiaire est inférieure ou égale à 20 %.

On notera que cette fraction massique des méthyl butènes dans cette coupe C5 intermédiaire (i.e. avant déshydrogénation) est extrêmement réduite par rapport aux fractions massiques en méthyl butènes proches de 100 % qui caractérisent les coupes C5 avant déshydrogénation utilisées dans les procédés connus d'enrichissement en isoprène des coupes de craquage catalytique sur lit fluide, pour la polymérisation sélective de l'isoprène avec un rendement élevé en vue de l'obtention d'un polyisoprène à fort taux d'enchaînements cis-1,4.

Selon un autre aspect de la présente invention, ladite coupe C5 intermédiaire est appauvrie en n-pentènes de telle sorte qu'elle comprenne lesdits n-pentènes selon une fraction massique inférieure à 0,1 %.

On notera que cet appauvrissement en n-pentènes de la coupe C5 intermédiaire (précurseurs des pentadiènes-1,3 et -1,4) permet d'éliminer pratiquement ces pentadiènes-1,3 et -1,4 de la coupe C5 finale.

En effet, selon un autre aspect de l'invention, la coupe C5 finale comprend du pentadiène-1,3 et du pentadiène-1,4 selon des rapports massiques (pentadiène-1,3/isoprène) et (pentadiène-1,4/ isoprène) qui doivent être inférieurs ou égaux à 0,5 % et 0,2 %, respectivement, pour pouvoir polymériser sélectivement l'isoprène à partir de cette coupe finale avec une cinétique de conversion satisfaisante qui soit proche de celle relative à la polymérisation isolée de l'isoprène (i.e. le milieu de polymérisation comprenant uniquement le solvant et l'isoprène) et obtenir un polyisoprène de viscosité élevée analogue à celle du polyisoprène obtenu isolément.

Selon une autre caractéristique de l'invention, ledit procédé d'obtention de ladite coupe C5 finale enrichie en isoprène comprend une réaction d'hydrogénation catalytique, telle qu'une hydrogénation catalytique au moyen d'un catalyseur à base de palladium, qui est appliquée à ladite coupe C5 initiale et qui produit ladite coupe C5 intermédiaire.

Selon un autre aspect de la présente invention, le rapport massique (méthyl butènes/ isoprène) dans ladite coupe C5 finale et purifiée est égal ou supérieur à 50 %.

Selon un autre aspect de l'invention, le rapport massique (mono-oléfines / isoprène) dans ladite coupe C5 finale et purifiée peut être avantageusement supérieur à 50 %.

Pour l'obtention de ladite coupe C5 finale et purifiée, la coupe C5 finale est soumise à des opérations de purification comprenant :

- une distillation sur de l'anhydride maléique pour éliminer pratiquement le cyclopentadiène résiduel,
- une élimination des alcynes vrais et des alcynes disubstitués par une distillation sur de l'hydrure de diisobutylaluminium (HDiBA), par une réaction d'hydrogénation catalytique sélective ou tout autre opération connue de l'homme de l'art, et
- en un passage sur de l'alumine ou sur un tamis moléculaire pour éliminer les impuretés polaires résiduelles.

On notera que la coupe C5 finale et ainsi purifiée comprend notamment, à titre de méthyl butènes, du méthyl-2 butène-2, et qu'elle comprend en outre des quantités minimes :

- d'alcynes disubstitués, le rapport massique (alcynes disubstitués/ isoprène) étant de préférence inférieur ou égal à 0,7 %,
- d'alcynes vrais, le rapport massique (alcynes vrais/ isoprène) dans la coupe C5 finale et purifiée étant de préférence inférieur ou égal à 15 ppm,
- de cyclopentadiène, le rapport massique (cyclopentadiène/ isoprène) dans cette coupe C5 finale et purifiée étant de préférence inférieur ou égal à 5 ppm.

La présente invention a également pour objet un procédé d'obtention d'un homopolymère d'isoprène présentant un taux d'enchaînements cis-1,4 égal ou supérieur à 98,0 %, qui est tel qu'il comprend la réaction dudit système catalytique selon l'invention dans un milieu de polymérisation comprenant de l'isoprène et au moins un méthyl butène, de manière que le rapport massique (méthyl butène(s)/ isoprène) soit égal ou supérieur à 50 % et avantageusement égal ou supérieur à 100 %.

On notera que cette réaction de polymérisation peut être mise en oeuvre dans un solvant hydrocarboné inerte, ou bien en masse, c'est-à-dire sans solvant.

Selon un autre aspect de l'invention relative à ce procédé d'obtention d'un homopolymère d'isoprène, le milieu de polymérisation comprend au moins une mono-oléfine et le rapport massique (mono-oléfine(s) / isoprène) est supérieur à 50 % dans ce milieu de polymérisation.

Selon une caractéristique préférentielle de ce procédé d'obtention d'un homopolymère d'isoprène selon l'invention, lesdits méthyl butènes comprennent le méthyl-2 butène-2.

A titre encore plus préférentiel, le rapport massique (méthyl-2 butène-2/isoprène) dans ledit milieu de polymérisation est égal ou supérieur à 20 % et, encore plus préférentiellement, égal ou supérieur à 50 %.

On notera que les coupes C5 de craquage catalytique sur lit fluide et lesdites coupes C5 finales et purifiées selon la présente invention qui en sont issues sont caractérisées en ce qu'elles comprennent toujours du méthyl-2 butène-2.

A titre encore plus préférentiel, lesdits méthyl butènes comprennent au moins le méthyl-2 butène-1 et le méthyl-2 butène-2, le rapport massique (méthyl-2 butène-1 / isoprène) et le rapport massique (méthyl-2 butène-2 / isoprène) étant chacun compris entre 20 et 60 %.

Selon un autre aspect de ce procédé d'obtention d'un homopolymère d'isoprène selon l'invention, la fraction massique d'isoprène dans ledit milieu de polymérisation est inférieure à 30 % et avantageusement inférieure ou égale à 10 %.

Selon un mode préférentiel de mise en oeuvre de ce procédé d'obtention d'un homopolymère d'isoprène selon l'invention, ledit procédé comprend les étapes suivantes :

(i) l'obtention, à partir d'une coupe C5 initiale de craquage catalytique sur lit fluide, d'une coupe C5 intermédiaire comprenant des méthyl butènes selon une fraction massique inférieure à 30 % et avantageusement inférieure ou égale à 20 %,

(ii) l'obtention d'une coupe C5 finale enrichie en isoprène et purifiée, utilisable pour former un milieu pour la polymérisation sélective de l'isoprène en présence dudit système catalytique, par une déshydrogénation appliquée à ladite coupe C5 intermédiaire, et

(iii) l'obtention dudit homopolymère d'isoprène par réaction de ladite coupe C5 finale enrichie et purifiée avec ledit système catalytique.

Selon un autre aspect de ce mode préférentiel dans lequel ledit milieu de polymérisation est formé par ladite coupe C5 finale et purifiée, le rapport massique (mono-oléfine(s) / isoprène) est supérieur à 50 % dans ce milieu de polymérisation.

Selon une autre caractéristique de ce mode préférentiel, ladite étape (i) comprend une hydrogénation catalytique, telle qu'une hydrogénation catalytique au moyen d'un catalyseur à base de palladium.

Selon un autre aspect de ce mode préférentiel, ladite coupe C5 intermédiaire est appauvrie en n-pentènes de telle sorte qu'elle comprenne lesdits n-pentènes selon une fraction massique inférieure à 0,1 %, et ladite coupe C5 finale comprend du pentadiène-1,3 et du pentadiène-1,4 selon des rapports massiques (pentadiène-1,3/ isoprène) et (pentadiène-1,4/ isoprène) qui doivent être respectivement inférieurs ou égaux à 0,5 % et à 0,2 % pour les raisons précitées.

Selon un autre aspect de ce mode préférentiel, ladite coupe C5 finale et purifiée comprend de l'isoprène selon une fraction massique inférieure à 30 % et avantageusement inférieure ou égale à 10 %.

Selon un autre aspect de ce mode préférentiel, le rapport massique (méthyl butènes/ isoprène) dans ladite coupe C5 finale et purifiée est égal ou supérieur à 50 %.

Selon un exemple préférentiel de mise en oeuvre dudit procédé d'obtention d'un homopolymère d'isoprène, on met en oeuvre la réaction de polymérisation de l'isoprène à une température inférieure ou égale à 5° C, pour que ledit homopolymère d'isoprène présente un taux d'enchaînements cis-1,4, mesuré selon les techniques de résonance magnétique nucléaire du carbone 13 ou de dosage par moyen infrarouge, qui varie de 99,0 % à 99,6 %.

On se reportera au document de brevet WO-A-02/38635 au nom des demanderesses pour une description détaillée de la mise en oeuvre de cette réaction à une température inférieure ou égale à 5° C.

Avantageusement, on obtient des polyisoprènes dont les taux d'enchaînements cis-1,4, mesurés par l'une ou l'autre des techniques précitées, sont égaux ou supérieurs à 99,3 % et appartiennent encore plus avantageusement à un domaine allant de 99,3 % à 99,6 %, lorsque la polymérisation est réalisée à une température allant de -55° C à -20° C.

A titre encore plus avantageux, on obtient des polyisoprènes dont les taux d'enchaînements cis-1,4, également mesurés par l'une ou l'autre des techniques précitées, sont égaux ou supérieurs à 99,5 % et sont par exemple égaux à 99,6 %, lorsque la polymérisation est réalisée à une température allant de -55° C à -45° C.

D'une manière générale, on notera que le taux particulièrement élevé d'enchaînements cis-1,4 obtenu pour les polyisoprènes selon l'invention est indépendant de la quantité utilisée dudit système catalytique.

Selon une autre caractéristique avantageuse de l'invention, les homopolymères d'isoprène qui sont obtenus par ledit procédé présentent des viscosités inhérentes, mesurées à 0,1 g/dl dans le toluène à 25° C, qui sont supérieures à 3 dl/g, avantageusement à 4 dl/g.

On notera que le système catalytique selon l'invention présente l'avantage de conférer, à la réaction de polymérisation de l'isoprène, pratiquement une même cinétique avantageuse de conversion de l'isoprène et, aux polyisoprènes obtenus, pratiquement les mêmes valeurs élevées de viscosité inhérente à des taux de conversion donnés, que le milieu de polymérisation comprenne ou pas lesdits méthyl butènes.

Selon une caractéristique préférentielle dudit système catalytique selon l'invention, le rapport molaire (agent d'alkylation/ sel de terre rare) appartient à un domaine allant de 1 à 2.

Selon une autre caractéristique préférentielle dudit système catalytique selon l'invention, ledit sel de terre rare est un tris[bis(2-éthylhexyl)phosphate] de terre(s) rare(s), tel que le tris[bis(2-éthylhexyl)phosphate] de néodyme.

Selon une autre caractéristique préférentielle de l'invention, ledit système catalytique vérifie l'une au moins des conditions suivantes :

(a) ledit système catalytique comprend ledit ou lesdits métaux de terre rare selon une concentration appartenant à un domaine allant de 0,01 à 0,06 mol/l,
(b) le rapport molaire (donneur d'halogène/ sel de terre rare) varie de 2,0 à 3,5,
(c) le rapport molaire (monomère diène conjugué / sel de terre rare) varie de 15 à 70,
(d) ledit monomère diène conjugué est le butadiène,
(e) ledit agent d'alkylation est l'hydrure de diisobutylaluminium, et
(f) ledit donneur d'halogène est le chlorure de diéthylaluminium.

A titre de monomère diène conjugué utilisable pour « préformer » le système catalytique selon l'invention, on peut citer le 1, 3-butadiène, à titre préférentiel.

On peut également citer le 2-méthyl 1, 3-butadiène (ou isoprène), les 2, 3-di (alcoyle en C1 à C5) 1, 3-butadiène tels que par exemple le 2, 3 diméthyl-1, 3-butadiène, le 2, 3-diéthyl-1, 3-butadiène, le 2-méthyl 3-éthyl 1, 3-butadiène, le 2-méthyl 3-isopropyl 1, 3-butadiène, le phényl 1, 3-butadiène, le 1, 3-pentadiène, le 2, 4-hexadiène, ou tout autre diène conjugué ayant entre 4 et 8 atomes de carbone.

Selon une autre caractéristique de l'invention, ledit sel de terre rare est constitué d'une poudre non hygroscopique ayant une légère tendance à s'agglomérer à la température ambiante.

Selon un mode préférentiel de réalisation de l'invention, le solvant hydrocarboné inerte dans lequel ledit sel de terre rare est en suspension est un solvant aliphatique ou alicyclique de bas poids moléculaire, tel que le cyclohexane, le méthylcyclohexane, le n-heptane, ou un mélange de ces solvants.

Selon un autre mode de réalisation de l'invention, le solvant utilisé pour la suspension du sel de terre rare est un mélange d'un solvant aliphatique de haut poids moléculaire comprenant une huile paraffinique, par exemple de l'huile de vaseline, et d'un solvant de bas poids moléculaire tel que ceux susmentionnés (par exemple le méthylcyclohexane).

On réalise cette suspension en procédant à un broyage dispersif du sel de terre rare dans cette huile paraffinique, de sorte à obtenir une suspension très fine et homogène du sel.

Comme indiqué ci-dessus, ledit système catalytique comprend le métal de terre rare selon une concentration appartenant à un domaine allant de 0,01 à 0,06 mol/l, par exemple égale ou sensiblement égale à 0,02 mol/ l.

A titre d'agent d'alkylation répondant à la formule $AlR_3$ ou $HAlR_2$ utilisable dans le système catalytique selon l'invention, on peut citer des alkylaluminiums tels que:

- des trialkylaluminiums, par exemple le triisobutylaluminium, ou
- des hydrures de dialkylaluminium, par exemple l'hydrure de diisobutylaluminium.

On notera que cet agent d'alkylation est de préférence constitué de l'hydrure de diisobutylaluminium (appelé HDiBA dans la suite de la présente description).

A titre de donneur d'halogène utilisable dans le système catalytique selon l'invention, on peut citer des halogénures d'alkylaluminium, de préférence le chlorure de diéthylaluminium (appelé CDEA dans la suite de la présente description). Comme indiqué ci-dessus, le rapport molaire (donneur d'halogène / sel de terre rare) peut présenter une valeur allant de 2,0 à 3,5.

Selon l'invention, le procédé de préparation dudit système catalytique consiste:

- dans une première étape, à réaliser une suspension dudit sel dans ledit solvant,
- dans une seconde étape, à ajouter à la suspension ledit monomère diène conjugué,
- dans une troisième étape, à ajouter ledit agent d'alkylation à la suspension comprenant ledit monomère pour l'obtention d'un sel alkylé, et
- dans une quatrième étape, à ajouter ledit donneur d'halogène au sel alkylé.

Les caractéristiques précitées de la présente invention, ainsi que d'autres, seront mieux comprises à la lecture de la description suivante de plusieurs exemples de réalisation de l'invention, donnés à titre illustratif et non limitatif.

**I. Préparation d'un système catalytique selon l'invention:**

**1) Synthèse d'un sel de phosphate organique de néodyme selon l'invention :**

a) Synthèse d'une solution aqueuse de néodyme $NdCl_3$, $6H_2O$ :

Dans un réacteur, on introduit une quantité donnée de $Nd_2O_3$. On ajoute 31,25 kg d'eau déminéralisée par kg de $Nd_2O_3$. On ajoute lentement, à température ambiante, 1,56 1 d'HCl concentré à 36 % en poids (d = 1,18) par kg de $Nd_2O_3$.

La réaction $Nd_2O_3 + 6\ HCl + 9\ H_2O \rightarrow 2\ NdCl_3$, $6H_2O$ est très exothermique.

Lorsque tout l'acide chlorhydrique a été ajouté, on porte la solution à ébullition sous agitation pendant 30 minutes, pour éliminer l'excès d'HCl. La solution aqueuse de $NdCl_3$ est limpide et de couleur mauve. Il ne reste pas de produit insoluble ($Nd_2O_3$).

Le pH de la solution, mesuré à 25°C, est corrigé par ajout de soude à 2 mole par litre. Le pH final est d'environ 4,5.

b) Synthèse d'un phosphate organique de sodium de formule $[RO]_2P(O)ONa$ (R=2-éthylhexyl):

Dans un réacteur vide on introduit 27,8 kg d'eau déminéralisée par kg de $Nd_2O_3$ de la synthèse du paragraphe a) ci-dessus. On dissout 0,708 kg de NaOH en pastilles par kg de $Nd_2O_3$ dudit paragraphe a). Dans un autre réacteur on ajoute, toujours par kg de $Nd_2O_3$ initial, 10,4 1 d'acétone et 5,819 kg d'un acide phosphorique organique (l'acide bis (2-éthylhexyl) phosphorique, répertorié dans l'ouvrage « Aldrich » sous la référence 23,782-5).

A température ambiante, on verse la solution dudit acide phosphorique organique dans la solution de NaOH. La réaction est la suivante :

$$[RO]_2P(O)OH + NaOH \rightarrow [RO]_2P(O)ONa + H_2O.$$

Cette réaction est légèrement exothermique, et l'on obtient une solution homogène de couleur claire. Le pH de la solution, mesuré à 25° C, est égal à 5,4.

c) Synthèse d'un sel phosphaté de néodyme de formule [[RO]$_2$P(O)O]$_3$Nd:

On verse sous vive agitation et à la température de 45° C la solution aqueuse de NdCl$_3$,6H$_2$O obtenue au paragraphe a) ci-dessus sur la solution de phosphate organique de Na obtenue au paragraphe b) ci-dessus. L'ajout peut, suivant les cas, être effectué dans l'ordre inverse. Il se forme immédiatement un précipité blanc très fin. On maintient le mélange obtenu sous agitation pendant 15 min., après l'addition de tout le phosphate organique de Na:

$3 [RO]_2P(O)ON_a + NdCl_3,6H_2O \rightarrow Nd[OP(O)[OR]_2]_3 + 3\ NaCl + 6\ H_2O.$

On récupère par sédimentation le sel phosphaté de néodyme ainsi obtenu et on le lave avec un mélange de 45 litres d'eau déminéralisée et 15 litres d'acétone pendant 15 minutes. Le sel phosphaté de néodyme est ensuite récupéré par centrifugation.

Le pH des eaux « mères » est compris entre 2 et 3 à 25° C. Ces eaux « mères » sont incolores et limpides. Sur la dernière eau de lavage, le test analytique qualitatif des chlorures est quasi-négatif (la réaction est: NaCl + AgNO$_3$ (milieu HNO$_3$) $\rightarrow$ AgCl $\downarrow$ + NaNO$_3$).

On sèche le sel de néodyme ainsi lavé dans une étuve à 60° C, sous vide et avec courant d'air pendant 72 heures.

**2) Synthèse d'un système catalytique « préformé » selon l'invention :**

a) Composition du système catalytique :

Le système catalytique comprend un sel phosphaté de néodyme tel que synthétisé selon le paragraphe 1) ci-dessus, qui est en suspension dans un solvant hydrocarboné inerte de bas poids moléculaire (constitué de méthylcyclohexane, « MCH » en abrégé ci-après).

Le système catalytique est caractérisé par les rapports molaires relatifs suivants, par rapport au sel de néodyme :

Sel de Nd / butadiène (Bd) / HDiBA / CDEA = 1 / 30 /1,8/2,6.

La concentration finale en Nd du système catalytique est de 0,02 M.

b) Procédé de synthèse du système catalytique:

- Première étape:

En vue de l'obtention du système catalytique, on verse 550 g de sel de néodyme, à l'état de poudre, dans un réacteur préalablement nettoyé de ses impuretés. On soumet ensuite ce sel à un barbotage à l'azote par le fond du réacteur, pendant 15 min.

- Seconde étape :

On introduit environ 90 % (fraction massique) du solvant mentionné au paragraphe 2)a) ci-dessus dans le réacteur contenant le sel de néodyme, la durée de mise en contact du sel de néodyme avec ce solvant étant de 30 min, la température de mise en contact étant de 30° C.

- Troisième étape :

On introduit ensuite du butadiène dans le réacteur (selon le rapport molaire sel / butadiène de 1/30 mentionné au paragraphe 2)a) ci-dessus) à la température de 30° C, en vue de la « préformation » du système catalytique.

- Quatrième étape :

On introduit ensuite dans le réacteur du HDiBA à titre d'agent d'alkylation du sel de néodyme, selon une concentration d'environ 1 M dans le MCH. La durée de l'alkylation est de 30 min. et la température de la réaction d'alkylation est de 30° C.

- Cinquième étape :

On introduit ensuite dans le réacteur du CDEA à titre de donneur d'halogène, selon une concentration d'environ 1 M dans le MCH. La température du milieu réactionnel est portée à 60˚ C.

- Sixième étape :

On procède ensuite à une « préformation » (ou vieillissement) du mélange ainsi obtenu en maintenant cette température de 60˚ C pendant une durée de 2 heures.

- Septième étape :

On obtient ainsi une solution de système catalytique. On procède à la vidange du réacteur et l'on transfère cette solution dans une bouteille "Steinie" de 750 ml, préalablement lavée, séchée et soumise à un barbotage à l'azote. On stocke finalement la solution catalytique sous atmosphère d'azote dans un congélateur, à la température de -15˚ C. Le tableau 1 ci-après contient les caractéristiques du système catalytique et de son procédé de préparation.

Tableau 1 :

|  | Système catalytique |
| --- | --- |
| Nd/Bd/HDiBA/CDEA | 1/30/1,8/2,6 |
| Solvatation (solvant/ durée/ température) | MCH 30 min. 30˚ C |
| Volume MCH (litres) | 20,5 |
| Masse phosphate de Nd (g) | 550 |
| Masse de butadiène (g) | 804 |
| Alkylation (durée, température) | 30 min. 30˚ C |
| Volume HDiBA (ml) | 1007 |
| Titre HDiBA (mol/l) | 0,89 |
| Vieillissement CDEA (durée, température) | 2 heures 60˚ C |
| Volume CDEA (ml) | 1310 |
| Titre CDEA (mol/l) | 0,985 |

## II. Essais «témoin» et selon l'invention de polymérisation de l'isoprène au moyen du système catalytique préparé au § I:

Le réacteur de polymérisation est une bouteille "Steinie" de 250 ml, qui contient 10,2 g d'isoprène et dont l'étanchéité est assurée par un ensemble de type "joint-capsule percée", permettant l'ajout dudit système catalytique selon l'invention à l'aide d'une seringue.

La polymérisation de l'isoprène est réalisée dans le cyclohexane à 50˚ C sous atmosphère inerte d'azote (le cyclohexane ayant été préalablement soumis à un barbotage à l'azote pendant 10 minutes).

Les essais « témoin » et selon l'invention ont été réalisés en utilisant 2,3 ml dudit système catalytique, soit une quantité de base catalytique en néodyme de 450 micromoles pour 100 grammes de monomère isoprène ($\mu$Mcm en abrégé).

De plus, on a utilisé pour ces essais un même rapport massique S/M (solvant/ monomère isoprène) qui est égal à 9 et une même fraction massique d'isoprène dans le milieu de polymérisation qui est égale à 10 %.

On a utilisé l'acétylacétone comme agent de stoppage de chaque réaction de polymérisation (1 ml d'une solution d'acétylacétone de concentration 1M dans le cyclohexane), et la N-1,3-diméthylbutyl-N'-phényl-p-phénylènediamine (6PPD en abrégé) comme agent de protection (selon une concentration de 0,4 pce).

Puis on a séché pendant environ 18 heures chaque solution extraite dans une étuve à 60˚ C sous vide (à une pression de 200 mm Hg), avec un léger courant d'azote.

La mesure du taux de conversion de l'isoprène en polyisoprène en fonction du temps de réaction décrit la cinétique de polymérisation pour chaque essai « témoin » et selon l'invention.

La viscosité inhérente à 0,1 g/dl dans le toluène, mesurée à 25˚C, caractérise la macrostructure de chaque

polyisoprène obtenu.

**1) <u>Essai de polymérisation « témoin » en l'absence de méthyl butène :</u>**

On a utilisé dans cet essai « témoin » de l'isoprène pratiquement pur ayant été extrait classiquement en laboratoire d'une coupe C5 de vapocraquage de naphta, en réalisant :

- une distillation de la coupe C5 initiale sur de l'anhydride maléique pour éliminer le cyclopentadiène résiduel, suivie
- d'un passage sur une colonne d'alumine pour éliminer les impuretés polaires, et
- d'un barbotage à l'azote pendant 10 min., juste avant la réaction de polymérisation.

On a déterminé, par la technique de chromatographie en phase gazeuse (CPG, voir annexe 3), la fraction massique de l'isoprène extrait de cette coupe C5, qui est de 99,2 %.
Pour cet essai « témoin », on a utilisé 15 ml d'isoprène (soit 10,2 g) et 128 ml de cyclohexane à titre de solvant de polymérisation, de telle sorte que la fraction massique d'isoprène dans le milieu de polymérisation soit sensiblement égale à 10 %.
Le tableau 2 ci-après détaille les résultats obtenus pour cet essai « témoin ».

Tableau 2 :

| Temps de polymérisation (min.) | Taux de conversion (%) | Viscosité inhérente (dl/g) |
|---|---|---|
| 10 | 32 | - |
| 20 | 68 | - |
| 30 | 86 | - |
| 60 | 104 | 4,26 |

**2) <u>Essai de polymérisation selon l'invention en présence de méthyl butènes :</u>**

On a utilisé dans cet essai selon l'invention un milieu de polymérisation comprenant environ 10 % d'isoprène, 5 % de méthyl-2 butène-1, 5 % de méthyl-2 butène-2 et 80 % de cyclohexane (fractions massiques).
On utilise dans cet essai 15 ml (soit 10,2 g) de l'isoprène préparé au § II. 1) ci-dessus (pur à environ 99,2 %) et 113 ml de cyclohexane.
Le méthyl-2 butène-1 utilisé est obtenu par purification, réalisée au moyen d'un passage goutte à goutte sur une colonne d'alumine, d'un méthyl-2 butène-1 pur à environ 95 % et commercialisé par la société Fluka. On utilise 7,8 ml de méthyl-2 butène-1, soit 5,1 g.
Le méthyl-2 butène-2 utilisé est obtenu par purification, réalisée au moyen d'un passage goutte à goutte sur une colonne d'alumine, d'un méthyl-2 butène-2 pur à environ 85 % de la société Fluka (ce méthyl-2 butène-2 a été dosé par chromatographie en phase gazeuse à 93 % de pureté). On utilise 7,7 ml de méthyl-2 butène-2, soit également 5,1 g.
Le tableau 3 ci-après détaille les résultats obtenus pour cet essai selon l'invention.

Tableau 3 :

| Temps polymérisation (min.) | Taux de conversion (%) | Viscosité inhérente (dl/g) |
|---|---|---|
| 10 | 25 | - |
| 20 | 57 | - |
| 30 | 77 | - |
| 60 | 101 | 4,42 |

**3) <u>Conclusions:</u>**

Les tableaux 2 et 3 montrent que le système catalytique selon l'invention, à base d'un monomère diène conjugué, d'un sel de terre rare d'un acide phosphorique organique en suspension dans un solvant hydrocarboné inerte et saturé, d'un agent d'alkylation constitué d'un alkylaluminium de formule $AlR_3$ ou $HAlR_2$ avec un rapport molaire (agent d'alkylation/ sel) allant de 1 à 5, et d'un donneur d'halogène constitué d'un halogénure d'alkylaluminium, permet de polymériser sélectivement l'isoprène en présence de méthyl-2 butène-1 et de méthyl-2 butène-2 avec un rapport massique (méthyl butènes/ isoprène) égal à 100 % (les rapports massiques (méthyl-2 butène-1/ isoprène) et (méthyl-2 butène-2/ isoprène) étant chacun de 50 %), pour l'obtention d'un polyisoprène présentant un taux d'enchaînements cis-1,4 égal ou supérieur à 98,0 % et une viscosité inhérente supérieure à 4,0 dl/g.

On notera que la cinétique de conversion de l'isoprène en polyisoprène relative à l'essai selon l'invention en présence de méthyl butènes est très proche de la cinétique relative à l'essai « témoin » sans méthyl butène. En outre, la viscosité inhérente du polyisoprène obtenu à l'essai selon l'invention est très proche de celle du polyisoprène obtenu à cet essai « témoin ».

**III. Obtention de coupes finales enrichies en isoprène conformes ou non à l'invention, à partir de coupes initiales de craquage catalytique sur lit fluide, pour la polymérisation sélective d'isoprène en présence du système catalytique du § I. :**

**1) Exemples d'hydrogénations de coupes initiales A et B pour l'obtention de coupes intermédiaires conformes ou non à invention, suivant divers taux d'hydrogénation :**

Dans cette première étape d'hydrogénation, on a soumis une coupe brute C5 initiale A ou B de craquage catalytique sur lit fluide (voir composition au tableau 4 ci-après) à une réaction d'hydrogénation, que l'on met en oeuvre de la manière suivante selon un exemple de réalisation de l'invention.

Dans une bouteille de 750 ml sont introduits 1 g de catalyseur à base de palladium et de carbonate de calcium (de formule $Pd/CaCO_3$ et contenant 5 % de Pd en poids) et 100 ml de la coupe C5 initiale. La bouteille étant capsulée sous une pression initiale de 4 bars d'hydrogène et étant agitée à 25° C, on ajuste la pression d'hydrogène à 4 bars toutes les heures pendant 3 heures. Le temps de réaction total est de 6 heures et 30 minutes.

Cette réaction d'hydrogénation a notamment pour effet d'hydrogéner les n-pentènes contenus dans la coupe initiale selon un taux d'hydrogénation T déterminé, de manière à réduire la quantité des n-pentènes dans la coupe hydrogénée intermédiaire en vue de la seconde étape de déshydrogénation qui a pour effet de transformer ces n-pentènes en pentadiènes-1,3 et -1,4.

a) Coupe initiale A et coupe intermédiaire A1 selon l'invention, hydrogénée selon un taux T1 :

Le tableau 4 ci-après détaille la composition de ladite coupe C5 initiale A et de la coupe C5 intermédiaire A1 obtenue suite à l'exemple ci-dessus de réaction d'hydrogénation selon un taux d'hydrogénation T1 (les compositions sont exprimées en fractions massiques dans la coupe correspondante en % ou en ppm, avec 1 ppm = 1 partie par million = $10^{-4}$ %).

Tableau 4 :

|  | Coupe C5 initiale A | Coupe C5 intermédiaire A1 (hydrogénation selon un taux T1) |
|---|---|---|
| Isoprène | 0,27 % | non déterminé |
| Isopentane | 41,62 % | 46,83 % |
| Pentane | 4,54 % | 24,92 % |
| Propène | non déterminé | non déterminé |
| Butène-1 | 0,04 % | < 100 ppm |
| Butène-2 (E) | 0,18 % | < 10 ppm |
| Butène-2 (Z) | 0,26 % | < 10 ppm |
| Pentène-2 (E) | 9,69 % | 750 ppm |
| Pentène-2 (Z) | 5,19 % | 143 ppm |
| Pentène-1 | 4,26 % | 70 ppm |
| Méthyl-2 butène-1 | 11,21 % | 1,28 % |
| Méthyl-3 butène-1 | 1,69% | 105 ppm |
| Méthyl-2 butène-2 | 16,39 % | 21,51 % |

(suite)

|  | Coupe C5 initiale A | Coupe C5 intermédiaire A1 (hydrogénation selon un taux T1) |
|---|---|---|
| Pentadiène-1,4- | 92 ppm | < 10 ppm |
| Pentadiène-1,3 (E+Z) | 0,30 % | 40 ppm |
| Cyclopentadiène | 0,15% | non déterminé |
| **Somme méthyl butènes** | **29,29 %** | **22,79 %** |
| **Somme n-pentènes** | **19,14 %** | **963 ppm** |

On notera que la coupe intermédiaire A1 obtenue, qui est destinée à être soumise à la réaction de déshydrogénation en vue de son enrichissement en isoprène, comprend une fraction massique de méthyl butènes (moins de 30 %) qui est très inférieure à la fraction massique proche de 100 % qui est usuellement requise pour la déshydrogénation, en vue d'obtenir une coupe finale utilisable pour l'homopolymérisation de l'isoprène.

On notera également que l'hydrogénation a permis d'appauvrir la coupe initiale A en n-pentène avant déshydrogénation de telle sorte que la coupe intermédiaire A1 comprenne moins de 0,1 % de n-pentènes, alors que ladite coupe initiale A en contenait près de 20 %.

b) Coupe initiale B et coupes intermédiaires B2 à B6 conformes ou non à l'invention :

On a soumis une autre coupe C5 initiale B similaire à la coupe C5 initiale A ci-dessus à des réactions d'hydrogénation mises en oeuvre d'une manière analogue et caractérisées par d'autres taux d'hydrogénation des n-pentènes T2 à T5, comme illustré au tableau 5 ci-après.

Tableau 5 :

|  | Coupe B initiale | Coupe B2 interméd.: taux T2 | Coupe B3 interméd.: taux T3 | Coupe B4 interméd.: taux T4 | Coupe B5 interméd.: taux T5 | Coupe B6 interméd.: taux T6 |
|---|---|---|---|---|---|---|
| Pentène-1 | 4,20 % | 0,06 % | 0,02 % | 80 ppm | 90 ppm | ≈ 59 ppm |
| Pentène-2 (E+Z) | 15,10 % | 4,40 % | 0,80 % | 0,34 % | 0,30 % | ≈ 9 ppm |
| Méthyl-2 buténe-1 | 11,20% | 1,70 % | 1,50% | 1,10 % | 1,10% | 0,70 % |
| Méthyl-3 butène-1 | 1,60 % | 0,01 % | 0,01 % | 50 ppm | 70 ppm | 38 ppm |
| Méthyl-2butène-2 | 16,70% | 24,60 % | 23,10 % | 22,70 % | 22,50 % | 17,70 % |
| isopentane | 41,10 % | 44,20 % | 45,40 % | 46,30 % | 46,90 % | 51,60 % |
| pentane | 4,60 % | 20,00 % | 23,60% | 24,30 % | 24,10 % | 24,70 % |
| **Somme méthyl butènes** | **29,50 %** | **26,31 %** | **24,61 %** | **23,81 %** | **23,61 %** | **18,40 %** |
| **Somme des n-pentènes** | **19,30%** | **4,46%** | **0,82 %** | **0,35 %** | **0,31%** | **≈68 ppm** |

On notera que chacune des coupes intermédiaires B2 à B6 obtenues, destinée à être soumise à une déshydrogénation en vue de son enrichissement en isoprène, comprend une fraction massique de méthyl butènes (moins de 30 %, voire moins de 20 % pour la coupe B6) très inférieure à la fraction massique proche de 100 % usuellement requise avant déshydrogénation, pour obtenir une coupe finale utilisable pour homopolymériser l'isoprène.

On notera également que l'hydrogénation selon les taux T2 à T5 n'a pas permis d'appauvrir la coupe initiale B en n-pentènes avant déshydrogénation de sorte que chaque coupe intermédiaire comprenne moins de 0,1% de n-pentènes. Par conséquent, les coupes intermédiaires B2 à B5 ne sont pas utilisables pour l'obtention de coupes finales selon l'invention comprenant au plus 0,5 % de pentadiène-1,3 et au plus 0,2 % de pentadiène-1,4, condition nécessaire pour pouvoir polymériser sélectivement l'isoprène avec une cinétique, de conversion satisfaisante proche de celle relative à la polymérisation isolée de l'isoprène et obtenir un polyisoprène de viscosité analogue à celle du polyisoprène obtenu isolément.

Par contre, l'hydrogénation de la coupe initiale B selon le taux T6 a permis d'appauvrir celle-ci en n-pentènes avant déshydrogénation, de sorte que la coupe intermédiaire B6 ainsi obtenue comprenne moins de 0,1 % de n-pentènes et soit donc conforme à l'invention.

**2) Exemples de déshydrogénation des coupes intermédiaires A1, B5 et B6 :**

Parmi les coupes intermédiaires hydrogénées A1 et B2 à B6, on a soumis les coupes intermédiaires A1, B5 et B6 à des déshydrogénations par exemple mises en oeuvre comme suit.

a) Déshydrogénation de la coupe intermédiaire A1 pour obtenir la coupe finale A1' selon l'invention:

Cette coupe intermédiaire A1 est introduite dans un réacteur contenant un lit catalytique de 140 ml chauffé à une température comprise entre 600˚ C et 700˚ C, sous des courants de vapeur d'eau et d'azote préchauffés à 650˚C.
Les débits de la coupe intermédiaire A1, de la vapeur d'eau et de l'azote sont respectivement de 1,63 ml/min., 1460 ml/min. et 3,76 ml/min.
Le lit catalytique est composé d'oxyde de fer, de chrome et de carbonate de potassium et il est commercialisé par SHELL sous le nom « Shell 105 ».
En sortie de réacteur et après condensation de la majorité de l'eau, les effluents passent au contact d'un « piège » de chlorure de calcium et d'un « piège » d'alumine, avant d'être condensés dans un bain de « carboglace » et d'acétone (à environ -60˚ C).
Le tableau 6 ci-après détaille la composition de la coupe finale A1' ainsi obtenue.

Tableau 6 :

|  | Coupe A initiale | Coupe A1 intermédiaire | Coupe A1' finale |
|---|---|---|---|
| Isoprène | 0,27% | non déterminé | 11,00% |
| Isopentane | 41,62 % | 46,83 % | 48,80% |
| Pentane | 4,54 % | 24,92 % | 25,40 % |
| Propène | non déterminé | non déterminé | 0,38 % |
| Butène-1 | 0,04 % | < 100 ppm | 1,48% |
| Butène-2 (E) | 0,18 % | < 10 ppm | 0,18 % |
| Butène-2 (Z) | 0,26 % | < 10 ppm | 0,14 % |
| Pentène-2 (E) | 9,69% | 750 ppm | 220 ppm |
| Pentène-2 (Z) | 5,19 % | 143 ppm | 130 ppm |
| Pentène-1 | 4,26 % | 70 ppm | 14.7 ppm |
| Méthyl-2 butène-1 | 11.21% | 1,28% | 2,53% |
| Méthyl-3 butène-1 | 1,69% | 105 ppm | 0,54 % |
| Méthyl-2 butène-2 | 16,39% | 21,51 % | 4,10 % |
| **Pentadiène-1,4** | **92 ppm** | **< 10 ppm** | **< 50 ppm** |
| **Pentadiène-1,3 (E+Z)** | **0.30%** | **40 ppm** | **530 ppm** |
| Cyclopentadiène | 0,15 % | non déterminé | 90 ppm |
| **Somme méthyl butènes** | **29,29%** | **22,79 %** | **7,17 %** |
| **Somme n-pentènes** | **19,14 %** | **963 ppm** | **497 ppm** |
| **Somme $\alpha$-oléfines** | **17,20 %** | **1,28 %** | **4,93 %** |

(suite)

| | Coupe A initiale | Coupe A1 intermédiaire | Coupe A1' finale |
|---|---|---|---|
| **Somme β-oléfines** | **31,71 %** | **21,51 %** | **4,42 %** |
| **Somme mono-oléfines** | **48,91 %** | **22,79 %** | **9,35 %** |

On notera que la coupe finale A1' est notamment caractérisée en ce qu'elle comprend :

- de l'isoprène selon une fraction massique pratiquement égale à 10 %,
- des méthyl butènes selon un rapport massique (méthyl butènes/ isoprène) de 65 %,
- des mono-oléfines selon un rapport massique (mono-oléfines/isoprène) de 85 %.

On notera également que l'étape d'hydrogénation, par la réduction significative du taux de n-pentènes qu'elle engendre, permet de minimiser le taux de pentadiènes dans la coupe finale A1'. En effet, les rapports massiques (pentadiène-1,3/ isoprène) et (pentadiène-1,4/ isoprène) sont respectivement inférieurs à 0,5 % et à 0,2 % dans la coupe finale A1', ce qui rend celle-ci utilisable après purification (destinée à éliminer les alcynes disubstitués, les alcynes vrais et le cyclopentadiène) pour la polymérisation sélective de l'isoprène.

**3) Polymérisation de l'isoprène contenu dans la coupe finale A'1 au moyen du système catalytique « préformé » selon l'invention :**

a) Purification de la coupe finale A'1 avant polymérisation :

On utilise 1100 ml de la coupe finale A'1 avant purification qui est soumise dans une première étape à une distillation sur anhydride maléique dans les conditions suivantes :

- colonne à distiller : 20 plateaux théoriques
- taux de reflux : 5
- anhydride maléique/cyclopentadiène : 30 (rapport molaire), soit 3 g d'anhydride maléique
- mise en contact de l'anhydride maléique et de la coupe : une nuit à température ambiante

et dans une seconde étape à une élution sur colonne d'alumine contenant 250-300 cm3 d'alumine régénérée, maintenue sous azote, avec deux passages successifs, goutte à goutte lent (100 ml/h).

b) Polymérisation de l'isoprène contenu dans cette coupe finale A'1 purifiée :

On procède à la polymérisation de la coupe finale A'1 contenant 3,42 g d'isoprène à l'aide du système catalytique « préformé » selon l'invention, décrit au § I-2)a), en opérant selon le procédé et les conditions décrites au § II, hormis le fait que l'on utilise une quantité de base catalytique en néodyme de 1170 micromoles pour 100 grammes de monomère isoprène et qu'on opère la polymérisation durant 220 minutes.

Le tableau 7 ci-après détaille les résultats obtenus pour cet essai conforme à l'invention.

Tableau 7 :

| Temps de polymérisation (min) | Taux de conversion (%) | Viscosité inhérente (dl/g) |
|---|---|---|
| 15 | 25 | |
| 30 | 40 | |
| 50 | 56 | |
| 80 | 72 | |
| 120 | 83 | |
| 220 | 97 | 4.86 |

Le taux de cis mesuré par analyse RMN du carbone 13 du polyisoprène est de 98.1% Indice de polydispersité mesuré par SEC : 2.6

c) Déshydrogénations de la coupe intermédiaire B5 pour obtenir trois coupes finales B5', B5" et B5''' non conformes à l'invention :

Le tableau 8 suivant détaille les compositions de trois coupes finales B5', B5", B5''' respectivement obtenues par des déshydrogénations appliquées à la coupe intermédiaire B5 précitée selon le § III. 2) a) ci-dessus, à trois températures de 625˚ C, 650˚ C et 675˚ C.

Tableau 8 :

| | Coupe B initiale | Coupe B5 intermédiaire : hydrogénation selon taux T5 | Coupe B5' finale : déshydrogén.à 625˚ C | Coupe B5" finale : déshydrogén.à 650˚ C | Coupe B5''' finale : déshydrogén.à 675˚ C |
|---|---|---|---|---|---|
| **isoprène** | | **non déterminé** | **13,00 %** | **12,60 %** | **12,60 %** |
| Pentène-1 | 4,20 % | 90 ppm | 0,04 % | 0,02 % | 0,03 % |
| Pentène-2 (E+Z) | 15,10 % | 0,30 % | 0,11 % | 0,08% | 0,08 % |
| **Pentadiène-1,4/ isoprène** | | | **0,06 %** | **0,06 %** | **0,06 %** |
| **Pentadiène-1,3/ isoprène** | | | **1,40 %** | **1,20 %** | **0,90 %** |
| Méthyl-2 butène-1 | 11,20 % | 1,10 % | 2,80 % | 2,30 % | 1,80 % |
| Méthyl-3 butène-1 | 1,60 % | 70 ppm | 0,50 % | 0,50 % | 0,30 % |
| Méthyl-2 butène-2 | 16,70 % | 22,50 % | 4,70 % | 3,70 % | 2,80 % |
| isopentane | 41,10% | 46,90 % | 44,60 % | 44,80 % | 44,70 % |
| pentane | 4,60 % | 24,10 % | 25,80 % | 24,40 % | 24,90 % |
| **Somme méthyl butènes** | **29,50 %** | **23,61 %** | **8,00 %** | **6,50 %** | **4,90 %** |
| **Somme n-pentènes** | **19,30%** | **0,31%** | **0,15 %** | **0,10%,** | **0,11%** |

On notera que chaque coupe finale B5', B5", B5''' comprend de l'isoprène selon une fraction massique inférieure à 15 % et que le rapport massique (méthyl butènes/ isoprène) à l'intérieur des coupes finales B5' et B5" est d'environ 60 % et 50 %, respectivement.

En outre, comme annoncé au § III. 2) b) ci-dessus, l'étape d'hydrogénation, par la réduction insuffisante du taux de n-pentènes qu'elle engendre, ne permet d'obtenir un rapport (pentadiène-1,3/ isoprène) dans les coupes finales B5', B5" et B5''' au plus égal à 0,5 %.

d) Déshydrogénations de la coupe intermédiaire B6 pour obtenir trois coupes finales B6', B6'' et B6''' conformes ou non à l'invention :

Le tableau 9 suivant détaille les compositions de coupes finales selon l'invention B6', B6", B6''' respectivement obtenues par des déshydrogénations appliquées à la coupe intermédiaire B6 selon le § III. 2) a) ci-dessus, à des températures de 600, 625 et 650˚ C.

Tableau 9 :

| | Coupe B initiale | Coupe B6 intermédiaire | Coupe B6' finale : déshydrogén.à 600˚ C | Coupe B6" finale : déshydrogén.à 625˚ C | Coupe B6''' finale : déshydrogén.à 650˚ C |
|---|---|---|---|---|---|
| **isoprène** | | **non déterminé** | **9,70 %** | **10,10 %** | **10,30 %** |
| Pentène- 1 | 4,20% | ≈ 59 ppm | 143 ppm | 133 ppm | 154 ppm |
| Pentène-2 (E+Z) | 15,10% | ≈ 9 ppm | 335 ppm | 358 ppm | 398 ppm |
| **pentadiène-1,4/ isoprène** | | | **0,02 %** | **0,03 %** | **0,03 %** |
| **pentadiène-1,3/ isoprène** | | | **0,50 %** | **0,60 %** | **0,70 %** |
| Méthyl-2 butène-1 | 11,20% | 0,70% | 2,50 % | 2,20 % | 1,50 % |
| Méthyl-3 butène- 1 | 1,60 % | 38 ppm | 0,50 % | 0,50 % | 0,30 % |
| Méthyl-2 butène-2 | 16,70 % | 17,70 % | 4,30 % | 3,70 % | 2,90 % |
| isopentane | 41,10% | 51,60 % | 50,10 % | 49,50 % | 48,90 % |
| pentane | 4,60 % | 24,70 % | 25,50 % | 25,30 % | 25,60 % |
| **Somme méthyl butènes** | **29,50 %** | **18,40 %** | **7,30 %** | **6,40 %** | **5,00 %** |
| **Somme n-pentènes** | **19,30 %** | **≈ 68 ppm** | **478 ppm** | **491 ppm** | **552 ppm** |
| **Somme α-oléfines** | **17,00 %** | **0,70 %** | **3,00 %** | **2,70 %** | **2,10 %** |
| **Somme β-oléfines** | **31,80 %** | **17,70 %** | **4,30 %** | **3,70 %** | **2,90 %** |
| **Somme mono-oléfines** | **48,80 %** | **18,40 %** | **7,30 %** | **6,40 %** | **5,00%** |

On notera que chaque coupe finale B6', B6" et B6'" est caractérisée en ce qu'elle comprend de l'isoprène selon une fraction massique pratiquement égale ou inférieure à 10 %.

On notera également que les coupes finales B6', B6" comprennent des méthyl butènes selon un rapport massique (méthyl butènes/ isoprène) pratiquement égal ou supérieur à 50 %.

Mais on notera que seule la coupe finale B6' (caractérisée par une température de déshydrogénation de 600˚ C) est utilisable pour polymériser sélectivement l'isoprène avec une cinétique de conversion satisfaisante proche de celle relative à la polymérisation isolée de l'isoprène et pour obtenir un polyisoprène de viscosité élevée analogue à celle du polyisoprène obtenu isolément, du fait que seule cette coupe B6' selon l'invention est caractérisée par un rapport (pentadiène-1,3/ isoprène) inférieur ou égal à 0,5 %.

**IV. Exemple comparatif non conforme à l'invention d'une coupe C' enrichie en isoprène par déshydrogénation, à partir d'une coupe initiale C de craquage catalytique sur lit fluide, sans hydrogénation de cette coupe initiale C :**

On a procédé directement à une déshydrogénation catalytique d'une coupe C5 initiale C de la manière décrite au § III. 2) a) ci-dessus, pour l'obtention d'une coupe C5 finale C enrichie en isoprène.

Le tableau 10 ci-après détaille les compositions respectives de ces coupes C et C'.

Tableau 10 :

|  | Coupe C5 initiale C | Coupe C5 deshydrogénée C' |
|---|---|---|
| Isoprène | 0,41 % | 15,05 % |
| Isopentane | 42,98 % | 40,61 % |
| Pentène-2 (E+Z) | 14,38% | 2,80% |
| Pentène- 1 | 4,86 % | 0,85% |
| Méthyl-2 butène-1 | 11,51% | 3,39% |
| Méthyl-3 butène-1 | 2,47 % | 0,78% |
| Méthyl-2 butène-2 | 14,41% | 5,24 % |
| **Pentadiène-1,4** | **0,02 %** | **0,21 %** |
| **Pentadiène-1,3 (E+Z)** | **0,39 %** | **4,6 %** |
| Cyclopentadiène | 0,21 % | 0,31 % |
| Somme méthyl butènes | 28,39% | 9,41 % |
| Somme n-pentènes | 19,24 % | 3,65 % |

On notera que l'absence d'hydrogénation préalable de la coupe initiale C avant l'étape de déshydrogénation conduit à l'obtention d'une fraction massique beaucoup trop élevée de pentadiène-1,3 dans la coupe C' déshydrogénée (4,6 % au lieu de la limite supérieure admissible selon l'invention de 0,5 %), ce qui ne permet pas de polymériser sélectivement l'isoprène à partir de cette coupe C' et au moyen du système catalytique du § I. avec une cinétique de conversion satisfaisante qui soit proche de celle relative à la polymérisation isolée de l'isoprène (i.e. le milieu de polymérisation comprenant uniquement le solvant cyclohexane et l'isoprène), ni d'obtenir un polyisoprène de viscosité élevée qui soit analogue à celle du polyisoprène obtenu isolément.

## ANNEXE 1 :

### Détermination de la microstructure des polyisoprènes.

### Par la technique de résonance magnétique nucléaire du carbone 13 (analyse RMN[13]C) :

a) Préparation des échantillons:

2 g de polyisoprène sont extraits à l'acétone au reflux pendant 8 heures. Le polyisoprène extrait est ensuite séché à température ambiante et sous vide pendant 24 heures. Puis ce polyisoprène séché est remis en solution dans du chloroforme. La solution de polyisoprène est filtrée et le solvant est éliminé à l'évaporateur rotatif pendant 4 heures (la température du bain est de 40˚ C).
Pour l'analyse, on solubilise environ 600 mg du polyisoprène ainsi préparé dans du $CDCl_3$ (2 ml), directement dans un tube RMN[13]C.

b) Caractéristiques de l'appareillage:

- Spectrophotomètre commercialisé sous la dénomination « BRUKER AM250 ».
- Fréquence de résonance (SFO) = 62,9 MHz.
- Programme d'impulsion : INVGATE.AU (suppression de l'effet « NOE » pour l'analyse quantitative en RMN du $^{13}$C).
- Durée d'impulsion : 9 $\mu$s (90˚).
- Durée de relaxation : 10 s.
- Nombre de transitoires accumulés (NS)= 8192.

c) Attribution des pics du spectre:

L'identification des pics a été faite d'après :

Quang Tho Pham, R. Petiaud, H. Waton, M.F. Llauro Darricades, *"Proton and NMR Spectra of Polymers"*, **1991**, Penton Press.

d) Méthode d'intégration:

- Pas de motifs 1-2 détectés.
- Le rapport entre les taux de 3-4 et de 1-4 est déterminé à l'aide des carbones éthyléniques. La teneur en enchaînements 1-4 trans et 1-4 cis dans le polyisoprène est calculée avec les carbones aliphatiques.

**ANNEXE 2 :**

**Détermination de la distribution des masses moléculaires des élastomères obtenus par la technique de chromatographie d'exclusion par la taille (SEC).**

a) Principe de la mesure:

La chromatographie d'exclusion par la taille ou SEC (size exclusion chromatography) permet de séparer physiquement les macromolécules suivant leur taille à l'état gonflé sur des colonnes remplies de phase stationnaire poreuse. Les macromolécules sont séparées par leur volume hydrodynamique, les plus volumineuses étant éluées en premier.

Sans être une méthode absolue, la SEC permet d'appréhender la distribution des masses moléculaires d'un polymère. A partir de produits étalons commerciaux, les différentes masses moyennes en nombre (Mn) et en poids (Mw) peuvent être déterminées et l'indice de polydispersité calculé (Ip = Mw/Mn).

b) Préparation du polymère:

Il n'y a pas de traitement particulier de l'échantillon de polymère avant analyse. Celui-ci est simplement solubilisé dans du tétrahydrofurane à une concentration d'environ 1 g/l.

c) Analyse SEC:

L'appareillage utilisé est un chromatographe « WATERS, modèle 150C ». Le solvant d'élution est le tétrahydrofurane, le débit de 0,7 ml/mm, la température, du système de 35°C et la durée d'analyse de 90 min. On utilise un jeu. de quatre colonnes en série, de dénominations commerciales «SHODEX KS807 », « WATERS type STYRAGEL HMW7 » et deux « WATERS STYRAGEL HMW6E».

Le volume injecté de la solution de l'échantillon de polymère est 100 $\mu$l. Le détecteur est un réfractomètre différentiel « WATERS modèle RI32X » et le logiciel d'exploitation des données chromatographiques est le système « WATERS MILLENIUM » (version 3.00).

**ANNEXE 3 :**

**Détermination des compositions des coupes C5 par chromatographie en phase gazeuse (CPG).**

a) Analyse CPG / FID :

L'analyse de chaque coupe C5 est effectuée à partir de volume injecté de 0,2 $\mu$l sans dilution préalable, afin de ne pas saturer la réponse du détecteur à ionisation de flamme (FID) qui est utilisé.

b) Conditions chromatographiques utilisées:

Chromatographe HP6890

(suite)

Gaz vecteur : azote

Débit constant : 0,7 ml/min.

Mode d'injection : « split »

Rapport de « split » : 50/1

Température de l'injecteur : 250˚ C

Volume injecté : 0,2 μl

| | |
|---|---|
| Colonne HP1: | phase 100 % de méthyl polysiloxane |
| | longueur : 60 m |
| | diamètre intérieur : 0,32 mm |
| | épaisseur du film : 1 μm |
| Programmation de température : | T1=15˚ C |
| | D1=20 min. |
| | P1=20˚ C/min. |
| | T2=280˚ C |
| | D2=4 min. |

Température du détecteur FID : 300˚ C.

c) Résultats;

On a réalisé une analyse semi-quantitative en calculant la proportion relative des aires des pics de chaque chromatogramme, pour obtenir une répartition. On n'a pas pris en compte les différences de réponses des composés élués, le détecteur FID ne détectant pas de signaux dus à la présence des composés non élués et élués.
La proportion en % d'un composé i est donnée par l'expression suivante:

$$\% \; i = A_i \, / \, \Sigma A_i \; x \; 100$$

avec

$A_i =$ aire relative au composé i, et
$\Sigma A_i =$ somme de tous les composés i élués (identifiés et non identifiés).

**Revendications**

1. Procédé d'obtention, à partir d'une coupe C5 initiale de craquage catalytique sur lit fluide, d'une coupe C5 finale enrichie en isoprène et purifiée qui est utilisable pour former un milieu pour la polymérisation sélective de l'isoprène en présence d'un système catalytique à base d'un diène conjugué, d'un sel de terre rare d'un acide phosphorique organique en suspension dans un solvant hydrocarboné inerte et saturé, d'un agent d'alkylation constitué d'un alkylaluminium de formule $AlR_3$ ou $HAlR_2$ dans laquelle Al représente un atome d'aluminium, H représente un atome d'hydrogène et les radicaux R, identiques ou différents, linéaires ou ramifiés, représentent des groupes hydrocarbonés ayant de 1 à 8 atomes de carbone, tel que le rapport molaire (agent d'alkylation/ sel) varie de 1 à 5, et d'un donneur d'halogène constitué d'un halogénure d'alkylaluminium, **caractérisé en ce que** ledit procédé comprend :

   (i) une réaction d'hydrogénation catalytique de la coupe C5 initiale comprenant des méthyl butènes au moyen d'un catalyseur à base de palladium qui produit une coupe C5 intermédiaire comprenant des n-pentène selon une fraction massique inférieure à 0,1% et lesdits méthyl butènes selon une fraction massique inférieure à 30 %
   (ii) une réaction de déshydrogénation qui est appliquée à la coupe C5 intermédiaire, comprenant des méthyl butènes et qui produit ladite coupe C5 finale, et
   (iii) une purification de ladite coupe C5 finale ainsi obtenue pour l'obtention de ladite coupe C5 finale et purifiée qui soit pratiquement exempte d'alcynes disubstitués, d'alcynes vrais et de cyclopentadiène.

2. Procédé d'obtention selon la revendication 1 de ladite coupe C5 finale et purifiée à partir de ladite coupe C5 initiale, **caractérisé en ce que** la fraction massique desdits méthyl butènes dans ladite coupe C5 intermédiaire est inférieure ou égale à 20 %.

3. Procédé d'obtention selon la revendication 1 ou 2 de ladite coupe C5 finale et purifiée à partir de ladite coupe C5 initiale, **caractérisé en ce que** ladite coupe C5 finale et purifiée comprend de l'isoprène selon une fraction massique inférieure à 30 %.

4. Procédé d'obtention selon la revendication 3 de ladite coupe C5 finale et purifiée à partir de ladite coupe C5 initiale, **caractérisé en ce que** ladite coupe C5 finale et purifiée comprend de l'isoprène selon une fraction massique inférieure ou égale à 10 %.

5. Procédé d'obtention selon une des revendications précédentes de ladite coupe C5 finale et purifiée à partir de ladite coupe C5 initiale, **caractérisé en ce que** ladite coupe C5 finale et purifiée comprend du pentadiène-1,3 et du pentadiène-1,4 selon des rapports massiques (pentadiène-1,3/ isoprène) et (pentadiène-1,4/ isoprène) qui sont respectivement inférieurs ou égaux à 0,5 % et à 0,2 %.

6. Procédé d'obtention selon une des revendications précédentes de ladite coupe C5 finale et purifiée à partir de ladite coupe C5 initiale, **caractérisé en ce que** le rapport massique (méthyl butènes/ isoprène) est égal ou supérieur à 50 % dans ladite coupe C5 finale et purifiée.

7. Procédé d'obtention selon une des revendications précédentes de ladite coupe C5 finale et purifiée à partir de ladite coupe C5 initiale, ladite coupe finale et purifiée comprenant des mono-oléfines, **caractérisé en ce que** le rapport massique (mono-oléfines / isoprène) est supérieur à 50 % dans ladite coupe C5 finale et purifiée.

8. Procédé d'obtention d'un homopolymère d'isoprène présentant un taux d'enchaînements cis-1,4 égal ou supérieur à 98,0 %, **caractérisé en ce qu'**il comprend une étape de réaction dans le milieu de polymérisation, en présence, d'un système catalytique à base d'au moins :

   - un monomère diène conjugué,
   - un sel d'un ou plusieurs métaux de terre rare d'un acide phosphorique organique, ledit sel étant en suspension dans au moins un solvant hydrocarboné inerte, saturé et de type aliphatique ou alicyclique compris dans ledit système catalytique,
   - un agent d'alkylation constitué d'un alkylaluminium de formule $AlR_3$ ou $HAlR_2$, dans laquelle Al représente un atome d'aluminium, H représente un atome d'hydrogène et les radicaux R, identiques ou différents, linéaires ou ramifiés, représentent des groupes hydrocarbonés ayant de 1 à 8 atomes de carbone, le rapport molaire (agent d'alkylation/ sel de terre rare) allant de 1 à 5, et
   - un donneur d'halogène constitué d'un halogénure d'alkylaluminium,

   le milieu de polymérisation comprenant de l'isoprène et au moins un méthyl butène dans des quantités telles que le rapport massique (méthyl butène(s)/ isoprène) soit égal ou supérieur à 50 % et que la fraction massique de l'isoprène dans le milieu de polymérisation soit inférieure à 30%,

9. Procédé d'obtention d'un homopolymère d'isoprène selon la revendication 8, ledit milieu de polymérisation comprenant au moins une mono-oléfine, **caractérisé en ce que** le rapport massique (mono-oléfine(s) / isoprène) est supérieur à 50 % dans ledit milieu de polymérisation.

10. Procédé d'obtention d'un homopolymère d'isoprène selon la revendication 8 ou 9, **caractérisé en ce que** ledit ou lesdits méthyl butènes comprennent le méthyl-2 butène-2.

11. Procédé d'obtention d'un homopolymère d'isoprène selon la revendication 10, **caractérisé en ce que** lesdits méthyl butènes comprennent le méthyl-2 butène-1 et le méthyl-2 butène-2, le rapport massique (méthyl-2 butène-1 / isoprène) et le rapport massique (méthyl-2 butène-2 / isoprène) étant chacun compris entre 20 % et 60 %.

12. Procédé d'obtention d'un homopolymère d'isoprène selon la revendication 8, **caractérisé en ce que** la fraction massique d'isoprène dans ledit milieu de polymérisation est inférieure ou égale à 10 %.

13. Procédé d'obtention d'un homopolymère d'isoprène selon une des revendications 8 à 12, **caractérisé en ce qu'**il comprend les étapes suivantes :

(i) une hydrogénation catalytique de la coupe C5 initiale comprenant des méthyl butènes au moyen d'un catalyseur à base de palladium qui produit une coupe C5 intermédiaire des méthyl butènes selon une fraction massique inférieure à 30%,

(ii) une déshydrogénation qui est appliquée à la coupe C5 intermédiaire, comprenant des méthyl butènes et qui produit ladite coupe C5 finale,

(iii) une purification de ladite coupe C5 finale ainsi obtenue pour l'obtention de ladite coupe C5 finale et purifiée qui soit pratiquement exempte d'alcynes disubstitués, d'alcynes vrais et de cyclopentadiène et

(iv) l'obtention dudit homopolymère d'isoprène par réaction de ladite coupe C5 finale et purifiée utilisée pour former le milieu pour la polymérisation sélective de l'isoprène en présence du système catalytique.

14. Procédé d'obtention d'un homopolymère d'isoprène selon la revendication 13, **caractérisé en ce que** la fraction massique desdits méthyl butènes dans ladite coupe C5 intermédiaire est inférieure ou égale à 20 %.

15. Procédé d'obtention d'un homopolymère d'isoprène selon la revendication 13, **caractérisé en ce que** ladite coupe C5 finale et purifiée comprend de l'isoprène selon une fraction massique inférieure ou égale à 10 %.

16. Procédé d'obtention d'un homopolymère d'isoprène selon la revendication13, **caractérisé en ce que** ladite coupe C5 intermédiaire est appauvrie en n-pentènes de telle sorte qu'elle comprenne lesdits n-pentènes selon une fraction massique inférieure à 0,1 %.

17. Procédé d'obtention d'un homopolymère d'isoprène selon une des revendications 13 à 16, **caractérisé en ce que** ladite coupe C5 finale et purifiée comprend du pentadiène-1,3 et du pentadiène-1,4 selon des rapports massiques (pentadiène-1,3/ isoprène) et (pentadiène-1,4/ isoprène) qui sont respectivement inférieurs ou égaux à 0,5 % et à 0,2 %.

18. Procédé d'obtention d'un homopolymère d'isoprène selon une des revendications 13 à 17, **caractérisé en ce que** le rapport massique (mono-oléfines / isoprène) est supérieur à 50 % dans ladite coupe C5 finale et purifiée.

19. Procédé d'obtention d'un homopolymère d'isoprène selon une des revendications 13 à 18, **caractérisé en ce que** l'étape de purification de ladite coupe C5 finale comprend une élimination des alcynes disubstitués jusqu'à l'obtention de ladite coupe C5 finale et purifiée comprenant lesdits alcynes disubstitués selon un rapport massique (alcynes disubstitués/ isoprène) inférieur ou égal à 0,7 %.

20. Procédé d'obtention d'un homopolymère d'isoprène selon une des revendications 8 à 19, **caractérisé en ce que** l'on met en oeuvre la réaction de polymérisation de l'isoprène à une température inférieure ou égale à 5° C, pour que ledit homopolymère d'isoprène présente un taux d'enchaînements cis-1,4, mesuré selon les techniques de résonance magnétique nucléaire du carbone 13 ou de dosage par moyen infrarouge, qui varie de 99,0 % à 99,6 %.

21. Procédé d'obtention d'un homopolymère d'isoprène selon une des revendications 8 à 20, **caractérisé en ce que** ledit homopolymère d'isoprène présente une viscosité inhérente, mesurée à 0,1 g/dl dans le toluène à 25° C, qui est supérieure à 4 dl/g.

22. Procédé d'obtention d'un homopolymère d'isoprène selon une des revendications 8 à 21, **caractérisé en ce que** dans ledit système catalytique, ledit sel de terre rare est un tris[bis(2-éthylhexyl)phosphate] de terre(s) rare(s), tel que le néodyme.

23. Procédé d'obtention d'un homopolymère d'isoprène selon une des revendications 8 à 22, **caractérisé en ce que** ledit système catalytique vérifie l'une au moins des conditions :

(a) ledit système catalytique comprend ledit ou lesdits métaux de terre rare selon une concentration appartenant à un domaine allant de 0,01 à 0,06 mol/l,
(b) le rapport molaire (donneur d'halogène/ sel de terre rare) varie de 2,0 à 3,5,
(c) le rapport molaire (monomère diène conjugué / sel de terre rare) varie de 15 à 70,
(d) ledit monomère diène conjugué est le butadiène,
(e) ledit agent d'alkylation est l'hydrure de diisobutylaluminium, et
(f) ledit donneur d'halogène est le chlorure de diéthylaluminium.

**Claims**

1.  A process for obtaining, from a fluid catalytically cracked initial C5 fraction, a final C5 fraction which is enriched with isoprene and purified which is usable to form a medium for the selective polymerisation of isoprene in the presence of a catalytic system based on a conjugated diene, a rare earth salt of an organic phosphoric acid suspended in a saturated inert hydrocarbon solvent, an alkylating agent consisting of an alkylaluminium of formula $AlR_3$ or $HAlR_2$ in which Al represents an aluminium atom, H represents a hydrogen atom and the radicals R, which may be identical or different, and straight-chain or branched, represent hydrocarbon groups having from 1 to 8 carbon atoms, such that the molar ratio (alkylating agent : salt) varies from 1 to 5, and a halogen donor consisting of an alkylaluminium halide, **characterised in that** it comprises:

    - a catalytic hydrogenation reaction of said initial C5 fraction comprising methyl butenes by means of a palladium-based catalyst which produces an intermediate C5 fraction comprising n-pentenes in a mass fraction of less than 0.1% and methyl butenes in a mass fraction of less than 30%,
    - a dehydrogenation reaction of said intermediate C5 fraction, comprising methyl butenes and which produces said final C5 fraction, and
    - purification of said final C5 fraction thus obtained to obtain said purified final C5 fraction which is practically devoid of disubstituted alkynes, true alkynes and cyclopentadiene.

2.  A process according to claim 1 for obtaining said purified final C5 fraction from said initial C5 fraction, **characterised in that** the mass fraction of said methyl butenes in said intermediate C5 fraction is less than or equal to 20%.

3.  A process according to claim 1 or 2 for obtaining said purified final C5 fraction from said initial C5 fraction, **characterised in that** said purified final C5 fraction comprises isoprene in a mass fraction of less than 30%.

4.  A process according to claim 3 for obtaining said purified final C5 fraction from said initial C5 fraction, **characterised in that** said purified final C5 fraction comprises isoprene in a mass fraction of less than or equal to 10%.

5.  A process according to one of the preceding claims for obtaining said purified final C5 fraction from said initial C5 fraction, **characterised in that** said purified final C5 fraction comprises 1,3-pentadiene and 1,4-pentadiene in mass ratios (1,3-pentadiene : isoprene) and (1,4-pentadiene : isoprene) which are less than or equal to 0.5% and 0.2% respectively.

6.  A process according to one of the preceding claims for obtaining said purified final C5 fraction from said initial C5 fraction, **characterised in that** the mass ratio (methyl butenes : isoprene) is greater than 50% in said purified final C5 fraction.

7.  A process according to one of the preceding claims for obtaining said purified final C5 fraction from said initial C5 fraction, said purified final fraction comprising mono-olefins, **characterised in that** the mass ratio (mono-olefins : isoprene) is greater than 50% in said purified final C5 fraction.

8.  A process for obtaining an isoprene homopolymer having a cis-1,4 linkage content equal to or greater than 98.0%, **characterised in that** it comprises a reaction step, in a polymerisation medium, in the presence of a catalytic system based on at least:

    - a conjugated diene monomer,
    - a salt of one or more rare earth metals of an organic phosphoric acid, said salt being suspended in at least one saturated inert hydrocarbon solvent of aliphatic or alicyclic type which is included in said catalytic system,
    - an alkylating agent consisting of an alkylaluminium of formula $AlR_3$ or $HAlR_2$, in which Al represents an aluminium atom, H represents a hydrogen atom and the radicals R, which may be identical or different, and straight-chain or branched, represent hydrocarbon groups having from 1 to 8 carbon atoms, the molar ratio (alkylating agent : rare earth salt) ranging from 1 to 5, and
    - a halogen donor consisting of an alkylaluminium halide, said polymerisation medium comprising isoprene and at least one methyl butene in quantities such that the mass ratio (methyl butene(s) : isoprene) is greater than 50% and that the mass fraction of isoprene in the polymerisation medium is less than 30%.

9.  A process for obtaining an isoprene homopolymer according to claim 8, said polymerisation medium comprising at least one mono-olefin, **characterised in that** the mass ratio (mono-olefin(s) : isoprene) is greater than 50% in said polymerisation medium.

**10.** A process for obtaining an isoprene homopolymer according to claim 8 or 9, **characterised in that** said methyl butene(s) comprise(s) 2-methyl 2-butene.

**11.** A process for obtaining an isoprene homopolymer according to claim 10, **characterised in that** said methyl butenes comprise 2-methyl 1-butene and 2-methyl 2-butene, the mass ratio (2-methyl 1-butene : isoprene) and the mass ratio (2-methyl 2-butene : isoprene) each being of between 20% and 60%.

**12.** A process for obtaining an isoprene homopolymer according to claim 8, **characterised in that** the mass fraction of isoprene in said polymerisation medium is less than or equal to 10%.

**13.** A process for obtaining an isoprene homopolymer according to one of claims 8 to 12, **characterised in that** it comprises the following stages:

(i) catalytic hydrogenation by means of a palladium-based catalyst of a fluid catalytically cracked initial C5 fraction to form an intermediate C5 fraction comprising methyl butenes in a mass fraction of less than 30%,
(ii) dehydrogenation of said intermediate C5 fraction in order to obtain a final C5 fraction enriched in isoprene which is usable to form a medium for the selective polymerisation of isoprene in the presence of said catalytic system, and
(iii) purification of said final C5 fraction to obtain a C5 fraction which is enriched in isoprene and purified which is practically devoid of disubstituted alkynes, true alkynes and cyclopentadiene, and the reaction of said purified final C5 fraction with said catalytic system.

**14.** A process for obtaining an isoprene homopolymer according to claim 13, **characterised in that** the mass fraction of said methyl butenes in said intermediate C5 fraction is less than or equal to 20%.

**15.** A process for obtaining an isoprene homopolymer according to claim 14, **characterised in that** said purified final C5 fraction comprises isoprene in a mass fraction of less than or equal to 10%.

**16.** A process for obtaining an isoprene homopolymer according to claim 13 or 14, **characterised in that** said intermediate C5 fraction is depleted in n-pentenes such that it comprises said n-pentenes in a mass fraction of less than 0.1%.

**17.** A process for obtaining an isoprene homopolymer according to one of claims 13 to 14, **characterised in that** said purified final C5 fraction comprises 1,3-pentadiene and 1,4-pentadiene in mass ratios (1,3-pentadiene : isoprene) and (1,4-pentadiene : isoprene) which are respectively less than or equal to 0.5% and to 0.2%.

**18.** A process for obtaining an isoprene homopolymer according to one of claims 13 to 17, **characterised in that** the mass ratio (mono-olefins : isoprene) is greater than 50% in said purified final C5 fraction.

**19.** A process for obtaining an isoprene homopolymer according to one of claims 13 to 18, **characterised in that** it furthermore comprises a stage of purification of said final C5 fraction, to obtain said purified final C5 fraction comprising disubstituted alkynes, in a mass ratio (disubstituted alkynes : isoprene) in said purified C5 fraction of less than or equal to 0.7%.

**20.** A process for obtaining an isoprene homopolymer according to one of claims 8 to 19, **characterised in that** the polymerisation reaction of the isoprene is effected at a temperature less than or equal to 5˚C, so that said isoprene homopolymer has a cis-1,4 linkage content, measured according to the techniques of carbon 13 nuclear magnetic resonance or of mid-infrared analysis, which varies from 99.0% to 99.6%.

**21.** A process for obtaining an isoprene homopolymer according to one of claims 8 to 20, **characterised in that** said isoprene homopolymer has an inherent viscosity, measured at 0.1 g/dl in toluene at 25˚C, which is greater than 4 dl/g.

**22.** A process for obtaining an isoprene homopolymer according to one of claims 8 to 21, **characterised in that** in said catalytic system, said rare earth salt is a tris[bis(2-ethylhexyl)phosphate] of rare earth(s), such as neodymium.

**23.** A process for obtaining an isoprene homopolymer according to one of claims 8 to 22, **characterised in that** said catalytic system satisfies at least one of the conditions:

(a) said catalytic system comprises said rare earth metal(s) in a concentration which lies within a range of from 0.01 to 0.06 mol/l,
(b) the molar ratio (halogen donor : rare earth salt) varies from 2.0 to 3.5,
(c) the molar ratio (conjugated diene monomer : rare earth salt) varies from 15 to 70,
(d) said conjugated diene monomer is butadiene,
(e) said alkylating agent is diisobutylaluminium hydride, and
(f) said halogen donor is diethylaluminium chloride.

**Patentansprüche**

1. Verfahren zur Herstellung eines isoprenangereicherten und gereinigten C5-Endschnitts, der zur Bildung eines Mediums zur selektiven Polymerisation von Isopren in Gegenwart eines katalytischen Systems auf Basis eines konjugierten Diens, eines Seltenerdmetallsalzes einer organischen Phosphorsäure in Suspension in einem inerten und gesättigten Kohlenwasserstoff-Lösungsmittel, eines aus einem Alkylaluminium der Formel $AlR_3$ oder $HAlR_2$, worin Al für ein Aluminiumatom steht, H für ein Wasserstoffatom steht und die Reste R gleich oder verschieden und linear oder verzweigt sind und für Kohlenwasserstoffgruppen mit 1 bis 8 Kohlenstoffatomen stehen, bestehenden Alkylierungsmittels bei einem Molverhältnis (Alkylierungsmittel/Salz) im Bereich von 1 bis 5 und eines aus einem Alkylaluminiumhalogenid bestehenden Halogendonors verwendbar ist, aus einem C5-Ausgangsschnitt aus dem katalytischen Cracken in der Wirbelschicht, **dadurch gekennzeichnet, daß** das Verfahren folgendes umfaßt:

(i) eine katalytische Hydrierungsreaktion des Methylbutene enthaltenden C5-Ausgangsschnitts mit Hilfe eines Palladiumkatalysators, die einen C5-Zwischenschnitt ergibt, der n-Pentene in einem Massenanteil von weniger als 0,1% und Methylbutene in einem Massenanteil von weniger als 30% enthält,
(ii) eine Dehydrierungsreaktion, die auf den Methylbutene enthaltenden C5-Zwischenschnitt angewandt wird und den C5-Endschnitt ergibt, und
(iii) eine Reinigung des so erhaltenen C5-Endschnitts, die den gereinigten C5-Endschnitt ergibt, der praktisch frei von disubstituierten Alkinen, echten Alkinen und Cyclopentadien ist.

2. Verfahren nach Anspruch 1 zur Herstellung des gereinigten C5-Endschnitts aus dem C5-Ausgangsschnitt, **dadurch gekennzeichnet, daß** der Massenanteil der Methylbutene in dem C5-Zwischenschnitt kleiner gleich 20% ist.

3. Verfahren nach Anspruch 1 oder 2 zur Herstellung des gereinigten C5-Endschnitts aus dem C5-Ausgangsschnitt, **dadurch gekennzeichnet, daß** der gereinigte C5-Endschnitt Isopren in einem Massenanteil von weniger als 30% enthält.

4. Verfahren nach Anspruch 3 zur Herstellung des gereinigten C5-Endschnitts aus dem C5-Ausgangsschnitt, **dadurch gekennzeichnet, daß** der gereinigte C5-Endschnitt Isopren in einem Massenanteil kleiner gleich 10% enthält.

5. Verfahren nach einem der vorhergehenden Ansprüche zur Herstellung des gereinigten C5-Endschnitts aus dem C5-Ausgangsschnitt, **dadurch gekennzeichnet, daß** der gereinigte C5-Endschnitt 1,3-Pentadien und 1,4-Pentadien in Massenverhältnissen (1,3-Pentadien/Isopren) und (1,4-Pentadien/Isopren) von kleiner gleich 0,5% bzw. 0,2% enthält.

6. Verfahren nach einem der vorhergehenden Ansprüche zur Herstellung des gereinigten C5-Endschnitts aus dem C5-Ausgangsschnitt, **dadurch gekennzeichnet, daß** das Massenverhältnis (Methylbutene/Isopren) in dem gereinigten C5-Endschnitt größer gleich 50% ist.

7. Verfahren nach einem der vorhergehenden Ansprüche zur Herstellung des gereinigten C5-Endschnitts aus dem C5-Ausgangsschnitt, wobei der gereinigte Endschnitt Monoolefine enthält, **dadurch gekennzeichnet, daß** das Massenverhältnis (Monoolefine/Isopren) in dem gereinigten C5-Endschnitt größer gleich 50% ist.

8. Verfahren zur Herstellung eines Isoprenhomopolymers mit einem Gehalt an cis-1,4-Verknüpfungen größer gleich 98,0%, **dadurch gekennzeichnet, daß** es einen Reaktionsschritt in einem Polymerisationsmedium in Gegenwart eines katalytischen Systems auf Basis von mindestens:

- einem konjugierten Dienmonomer,
- einem oder mehreren Seltenerdmetallsalzen einer organische Phosphorsäure, wobei das Salz in einem inerten, gesättigten Kohlenwasserstoff-Lösungsmittel vom aliphatischen oder alicyclischen Typ, das in dem katalytischen System enthalten ist, suspendiert ist,
- einem aus einem Alkylaluminium der Formel $AlR_3$ oder $HAlR_2$, worin Al für ein Aluminiumatom steht, H für ein Wasserstoffatom steht und die Reste R gleich oder verschieden und linear oder verzweigt sind und für Kohlenwasserstoffgruppen mit 1 bis 8 Kohlenstoffatomen stehen, bestehenden Alkylierungsmittel bei einem Molverhältnis (Alkylierungsmittel/Seltenerdmetallsalz) im Bereich von 1 bis 5 und
- einem aus einem Alkylaluminiumhalogenid bestehenden Halogendonor

umfaßt, wobei das Polymerisationsmedium Isopren und mindestens ein Methylbuten in solchen Mengen enthält, daß das Massenverhältnis (Methylbuten(e)/Isopren) größer gleich 50% ist und der Massenanteil an Isopren in dem Polymerisationsmedium unter 30% liegt.

9. Verfahren zur Herstellung eines Isoprenhomopolymers nach Anspruch 8, wobei das Polymerisationsmedium mindestens ein Monoolefin enthält, **dadurch gekennzeichnet, daß** das Massenverhältnis (Mono-olefin(e)/Isopren) in dem Polymerisationsmedium über 50% liegt.

10. Verfahren zur Herstellung eines Isoprenhomopolymers nach Anspruch 8 oder 9, **dadurch gekennzeichnet, daß** das oder die Methylbutene 2-Methyl-2-buten umfassen.

11. Verfahren zur Herstellung eines Isoprenhomopolymers nach Anspruch 10, **dadurch gekennzeichnet, daß** die Methylbutene 2-Methyl-1-buten und 2-Methyl-2-buten umfassen, wobei das Massenverhältnis (2-Methyl-1-buten/Isopren) und das Massenverhältnis (2-Methyl-2-buten/Isopren) jeweils zwischen 20 und 60% liegt.

12. Verfahren zur Herstellung eines Isoprenhomopolymers nach Anspruch 8, **dadurch gekennzeichnet, daß** der Massenanteil an Isopren in dem Polymerisationsmedium kleiner gleich 10% ist.

13. Verfahren zur Herstellung eines Isoprenhomopolymers nach einem der Ansprüche 8 bis 12, **gekennzeichnet durch** die folgenden Schritte:

(i) eine katalytische Hydrierung des Methylbutene enthaltenden C5-Ausgangsschnitts mit Hilfe eines Palladiumkatalysators, die einen C5-Zwischenschnitt ergibt, der Methylbutene in einem Massenanteil von weniger als 30% enthält,
(ii) eine Dehydrierung, die auf den Methylbutene enthaltenden C5-Zwischenschnitt angewandt wird und den C5-Endschnitt ergibt,
(iii) eine Reinigung des so erhaltenen C5-Endschnitts, die den gereinigten C5-Endschnitt ergibt, der praktisch frei von disubstituierten Alkinen, echten Alkinen und Cyclopentadien ist, und
(iv) die Herstellung des Isoprenhomopolymers durch Reaktion des zur Bildung des Mediums zur selektiven Polymerisation von Isopren in Gegenwart eines katalytischen Systems verwendeten gereinigten C5-Endschnitts.

14. Verfahren zur Herstellung eines Isoprenhomopolymers nach Anspruch 13, **dadurch gekennzeichnet, daß** der Massenanteil der Methylbutene in dem C5-Zwischenschnitt kleiner gleich 20% ist.

15. Verfahren zur Herstellung eines Isoprenhomopolymers nach Anspruch 13, **dadurch gekennzeichnet, daß** der gereinigte C5-Endschnitt Isopren in einem Massenanteil kleiner gleich 10% enthält.

16. Verfahren zur Herstellung eines Isoprenhomopolymers nach Anspruch 13, **dadurch gekennzeichnet, daß** der C5-Zwischenschnitt so sehr an n-Pentenen abgereichert wird, daß er die n-Pentene in einem Massenanteil unter 0,1% enthält.

17. Verfahren zur Herstellung eines Isoprenhomopolymers nach einem der Ansprüche 13 bis 16, **dadurch gekennzeichnet, daß** der gereinigte C5-Endschnitt 1,3-Pentadien und 1,4-Pentadien in Massenverhältnissen (1,3-Pentadien/Isopren) und (1,4-Pentadien/Isopren) von kleiner gleich 0,5% bzw. 0,2% enthält.

18. Verfahren zur Herstellung eines Isoprenhomopolymers nach einem der Ansprüche 13 bis 17, **dadurch gekennzeichnet, daß** das Massenverhältnis (Monoolefine/Isopren) in dem gereinigten C5-Endschnitt größer 50% ist.

**19.** Verfahren zur Herstellung eines Isoprenhomopolymers nach einem der Ansprüche 13 bis 18, **dadurch gekennzeichnet, daß** der Schritt der Reinigung des C5-Endschnitts eine Abtrennung der disubstituierten Alkine bis zum Erhalt des gereinigten C5-Endschnitts, der die disubstituierten Alkine in einem Massenverhältnis (disubstituierte Alkine/Isopren) kleiner gleich 0,7% enthält, umfaßt.

**20.** Verfahren zur Herstellung eines Isoprenhomopolymers nach einem der Ansprüche 8 bis 19, **dadurch gekennzeichnet, daß** man die Isoprenpolymerisationsreaktion bei einer Temperatur kleiner gleich 5°C durchführt, so daß das Isoprenhomopolymer einen durch die Techniken der Kohlenstoff-13-Kernresonanz oder quantitativen Mittelinfrarotanalyse bestimmten Gehalt an cis-1,4-Verknüpfungen von 99,0 bis 99,6% aufweist.

**21.** Verfahren zur Herstellung eines Isoprenhomopolymers nach einem der Ansprüche 8 bis 20, **dadurch gekennzeichnet, daß** das Isoprenhomopolymer eine bei einer Konzentration von 0,1 g/dl in Toluol bei 25°C gemessene inhärente Viskosität von mehr als 4 dl/g aufweist.

**22.** Verfahren zur Herstellung eines Isoprenhomopolymers nach einem der Ansprüche 8 bis 21, **dadurch gekennzeichnet, daß** es sich in dem katalytischen System bei dem Seltenerdmetallsalz um ein Tris-[bis (2-ethylhexyl) phosphat] von Seltenerdmetall(en) wie Neodym handelt.

**23.** Verfahren zur Herstellung eines Isoprenhomopolymers nach einem der Ansprüche 8 bis 22, **dadurch gekennzeichnet, daß** das katalytische System eine oder mehrere der folgenden Bedingungen erfüllt:

(a) das katalytische System enthält das oder die Seltenerdmetalle in einer Konzentration in einem Bereich von 0,01 bis 0,06 mol/l,
(b) das Molverhältnis (Halogendonor/Seltenerdmetallsalz) beträgt 2,0 bis 3,5,
(c) das Molverhältnis (konjugiertes Dienmonomer/Seltenerdmetallsalz) beträgt 15 bis 70,
(d) das konjugierte Dienmonomer ist Butadien,
(e) das Alkylierungsmittel ist Diisobutylaluminiumhydrid, und
(f) der Halogendonor ist Diethylaluminiumchlorid.

## RÉFÉRENCES CITÉES DANS LA DESCRIPTION

**Documents brevets cités dans la description**

- WO 0248218 A
- FR 1203754
- FR 2782996 A
- WO 0238635 A